# EUROPÄISCHE PATENTSCHRIFT

(11) **EP 0 455 080 B1**
(45) Veröffentlichungstag und Bekanntmachung des Hinweises auf die Patenterteilung: **20.12.1995**
(21) Anmeldenummer: 91106393.1
(22) Anmeldetag: 20.04.1991
(51) Int. Cl.: C07D 231/10, C07D 249/08, C07D 233/54, C07D 235/22, C07D 249/04, C07D 249/18, A01N 43/50, A01N 43/56, A01N 43/647, A01N 43/653

(54) **Biocide N-Thiocyanatomethoxy-azaheterocyclen**
Biocide N-thiocyanatomethoxy-azaheterocycles
N-thiocyanatométhoxy-azahétérocycles biocides

(30) Priorität: 30.04.1990 DE 4013874
(43) Veröffentlichungstag der Anmeldung: 06.11.1991
(73) Patentinhaber: BASF Aktiengesellschaft, 67063 Ludwigshafen (DE)
(72) Erfinder: Reuther, Wolfgang, Dr., W-6900 Heidelberg (DE); Baus, Ulf, Dr., W-6915 Dossenheim (DE); Lorenz, Gisela, Dr., W-6730 Neustadt (DE)

(56) Entgegenhaltungen:
- DE-A- 3 620 579
- DE-A- 3 740 285

## Beschreibung

Die vorliegende Erfindung betrifft neue N-Thiocyanatomethoxy-azaheterocyclen der Formel I

NCS-CH₂-O-Het I

in der Het die folgende Bedeutung hat:
- die 1,2,3-Triazol-1-ylgruppe oder die 1,2,4-Triazol-1-ylgruppe, oder
- die Indazol-1-ylgruppe, die Benzimidazol-1-ylgruppe oder die Benzotriazol-1-ylgruppe, wobei die annellierten Benzolringe dieser Substituenten noch jeweils bis zu drei der folgenden Reste tragen können: Halogen, C₁-C₄-Alkyl, C₁-C₄-Alkoxy oder partiell oder vollständig halogeniertes C₁-C₄-Alkyl,
sowie deren pflanzenverträgliche Salze und Metallkomplexe.

Außerdem betrifft die Erfindung Verfahren zur Herstellung dieser Verbindungen, ihre Verwendung als Biocide und biocide Mittel, welche diese Verbindungen als wirksame Substanzen enthalten.

Aus der DE-A 36 20 579 sind N-Thiocyanatomethoxy-pyrazol und Derivate dieser Verbindung, die am Pyrazolring durch Halogen substituiert sind, zur Bekämpfung von Mikroorganismen wie Bakterien, Pilzen und Algen bekannt.

Die Wirkungen dieser Verbindungen gegen Mikroorganismen können jedoch, besonders bei kleinen Aufwandmengen und -konzentrationen, nur bedingt befriedigen.

Der Erfindung lag daher die Aufgabe zugrunde, neue biocid wirksame N-Thiocyanatomethoxy-azaheterocyclen mit verbesserter Wirkung bereitzustellen.

Demgemäß wurden die eingangs definierten N-Thiocyanatomethoxy-azaheterocyclen der Formel I gefunden.

Im einzelnen hat der Azaheterocyclus in den erfindungsgemäßen Verbindungen I die folgende Bedeutung:
- die 1,2,3-Triazol-1-ylgruppe oder die 1,2,4-Triazol-1-ylgruppe,
- die Indazol-1-ylgruppe, die Benzimidazol-1-ylgruppe oder die Benztriazol-1-ylgruppe, wobei die annellierten Benzolringe dieser Substituenten noch jeweils bis zu drei der folgenden Reste tragen können:
   - Halogen, bevorzugt Fluor, Chlor und Brom,
   - verzweigtes oder unverzweigtes C₁-C₄-Alkyl wie Methyl, Ethyl, Propyl, Isopropyl, Butyl und tert.-Butyl, bevorzugt Methyl,
   - verzweigtes oder unverzweigtes C₁-C₄-Alkoxy wie Methoxy, Ethoxy, Propoxy und tert.-Butoxy, bevorzugt Methoxy,
   - partiell oder vollständig halogeniertes C₁-C₄-Alkyl, bevorzugt Trifluormethyl, Trichlormethyl, Pentafluorethyl und 2-Chlor-1,1,2-trifluormethoxy.

Besonders bevorzugt ist 1-Thiocyanatomethoxy-1,2,4-triazol.

Die N-Thiocyanatomethoxy-azaheterocyclen I sind auf verschiedene Weise erhältlich, und zwar vorzugsweise aus Verbindungen der Formel IIa

HO-Het IIa

nach folgenden Methoden:
a) Umsetzung mit Methylenbisthiocyanat 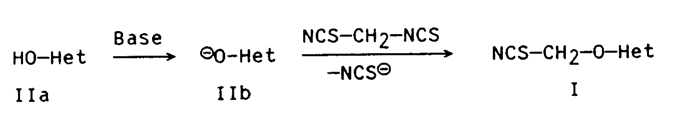 Die aus der DE-A 317 912 an sich bekannte Synthese wird in der Regel in inerten Lösungsmitteln, z.B. Ethern wie Diethylether, Tetrahydrofuran, Diethylenglykoldimethylether und aromatischen Kohlenwasserstoffen wie Toluol durchgeführt.
   Prinzipiell geeignet sind jedoch auch Lösungsmittelgemische aus Wasser und einem inerten, mit Wasser mischbaren oder unmischbaren organischen Lösungsmittel.
   Im ersten Reaktionsschritt wird der Heteroaromat IIa nach bekannten Verfahren mit einer basischen Alkalimetall- oder Erdalkalimetallverbindung, beispielsweise einem Alkalimetallhydroxid, Alkalimetallhydrid oder Alkalimetallcarbonat, in sein Alkoholat IIb umgewandelt. Bevorzugte Basen sind Lithium-, Natrium-, Kalium- und Magnesiumverbindungen wie Butyllithium, Natriumhydroxid, Natriumhydrid und Natriumcarbonat.
   Alkoholat und Methylenbisthiocyanat werden normalerweise in äquimolaren Mengen oder mit einem Überschuß der einen oder anderen Komponente bis etwa 10 % eingesetzt.
   Im allgemeinen liegt die Reaktionstemperatur zwischen 0 und 100°C, bevorzugt zwischen 10 und 70°C, insbesondere bei 20 bis 25°C (Raumtemperatur).
   Da die Reaktion nicht druckabhängig ist, empfiehlt sich das Arbeiten bei Normaldruck.
b) Umsetzung mit Chlormethylthiocyanat 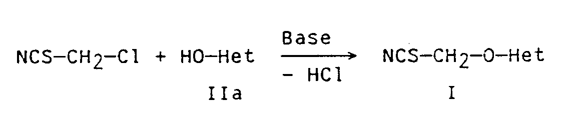 Die aus der DE-A 36 20 579 an sich bekannte Synthese erfolgt in der Regel in inerten Lösungsmitteln, beispielsweise aromatischen und aliphatischen Kohlenwasserstoffen oder Chlorkohlenwasserstoffen wie Toluol und Methylenchlorid, Ethern wie Diethylether, Tetrahydrofuran und Diethylenglykoldimethylether, Alkoholen wie tert.-Butanol, Ketonen wie Aceton, Nitrilen wie Acetonitril und Amiden wie N,N-Dimethylformamid.
   Als Basen eignen sich z.B. tertiäre Amine, Pyridin, Alkalicarbonate, Alkalihydroxide, Alkalihydride und Alkalialkoholate.
   Im allgemeinen liegt die Temperatur zwischen (-60) und 180°C, bevorzugt zwischen 20 und 80°C.
   Bezüglich der stöchiometrischen Verhältnisse und des Druckes gelten die Angaben für Methode a).
   Die Durchführung der Reaktion ist auch in einem 2-Phasensystem unter Phasentransfer-Katalyse möglich. Dazu kann vorteilhaft eine Mischung aus einem chlorierten Kohlenwasserstoff wie Methylenchlorid, wäßriger Lauge, z.B. Natronlauge, und Phasentransfer-Katalysator wie Tetra-n-butylammoniumhydroxid verwendet werden. In diesem Fall arbeitet man z.B. bei Temperaturen zwischen 10°C und der Siedetemperatur einer der Komponenten des Lösungsmittelgemisches.
c) Schrittweise Umsetzung mit einem Methandihalogenid und einem Alkalimetallthiocyanat 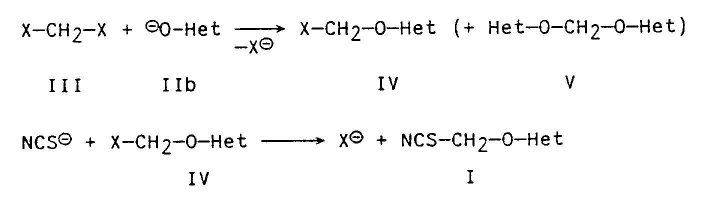 X = Cl, Br, J
   Die aus der US-PS 3 520 976 an sich bekannte Synthese erfolgt in der Regel in einem inerten Lösungsmittel, besonders bevorzugt in Aceton.
   Im ersten Reaktionsschritt wird das Alkoholat IIb mit einem Methandihalogenid, bevorzugt Bromchlormethan, zu dem Halogenmethylether IV umgesetzt, wobei als Nebenprodukt ein Acetal V des Formaldehyds entsteht.
   Um die Bildung des Nebenproduktes möglichst gering zu halten, empfiehlt sich ein Überschuß an Methandihalogenid III gegenüber dem Alkoholat, bis etwa zur 10fachen Menge.
   Im allgemeinen arbeitet man bei ca. 20°C (Raumtemperatur) oder bei leicht erhöhter Temperatur bis etwa 40°C.
   Die Umsetzung des Halogenmethylethers IV (rein oder verunreinigt mit Acetal V) mit einem Thiocyanat, beispielsweise einem Alkalimetall- oder bevorzugt mit Ammoniumthiocyanat, erfolgt zweckmäßig im stöchiometrischen Verhältnis oder mit einem Überschuß an Thiocyanat bis etwa 25 %.
   Zweckmäßigerweise arbeitet man bei einer Reaktionstemperatur zwischen ca. -20°C und dem Siedepunkt des Lösungsmittels.
   Bezüglich des Druckes gelten die Angaben für Methode a).

Die 1-Hydroxy-heteroverbindungen der Formel IIa sind bekannt oder nach bekannten Verfahren herstellbar. Synthesewege werden beispielsweise in folgenden Druckschriften beschrieben:
- für 1-Hydroxy-pyrazole: EP 347 676 und EP 347 689,
- für 1-Hydroxy-1,2,3-triazol: Ber. 27, 3381 (1894),
- für 1-Hydroxy-1,2,4-triazole: DE 39 00 347,
- für 1-Hydroxy-indazole: DE 25 22 314 und EP 347 676 und
- für 1-Hydroxy-benzimidazole: Synthesis, 703 (1975) und DE 39 32 552.
1-Hydroxy-benz-1,2,3-triazol ist kommerziell erhältlich.

Als Säureadditionssalze eignen sich die Salze von solchen Säuren, welche die fungizide Wirkung von I nicht beeinträchtigen, also z.B. die Hydrochloride und -bromide, Sulfate, Nitrate, Phosphate, Oxalate oder die Dodecylbenzolsulfonate.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und mineralsauren Salzen, beispielsweise den Chloriden oder Sulfaten, der Metalle her.

### Beispiel 1 (Herstellungsbeispiel)

### 3,5-Dimethyl-1-thiocyanatomethoxy-pyrazol

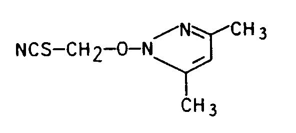

Zu einer Lösung von 11,2 g (0,1 mol) 3,5-Dimethyl-1-hydroxypyrazol in 350 ml Diethylenglykoldimethylether wurden bei ca. 20°C 11,2 g (0,1 mol) 50%ige wäßrige Kalilauge und anschließend etwa 15 g Molekularsieb 3 Å gegeben. Danach wurden in die Mischung 13,0 g (0,1 mol) Methylenbisthiocyanat eingerührt. Nach beendeter Zugabe wurde noch 4 Stunden lang bei Raumtemperatur (ca. 20°C) gerührt, dann das Molekularsieb abfiltriert und das Filtrat eingeengt. Nach Lösen des Rückstandes in Diethylketon und Waschen der organischen Phase mit wäßriger Natriumhydrogencarbonatlösung arbeitete man wie üblich auf das Produkt hin auf. Ausbeute: 51 %.

Der schwachgelbe Feststoff wurde aus Cyclohexan umkristallisiert.

Die physikalischen Daten der Verbindung sind Tabelle A zu entnehmen, in der weitere Verbindungen I aufgeführt sind, welche auf die gleiche Weise hergestellt wurden.

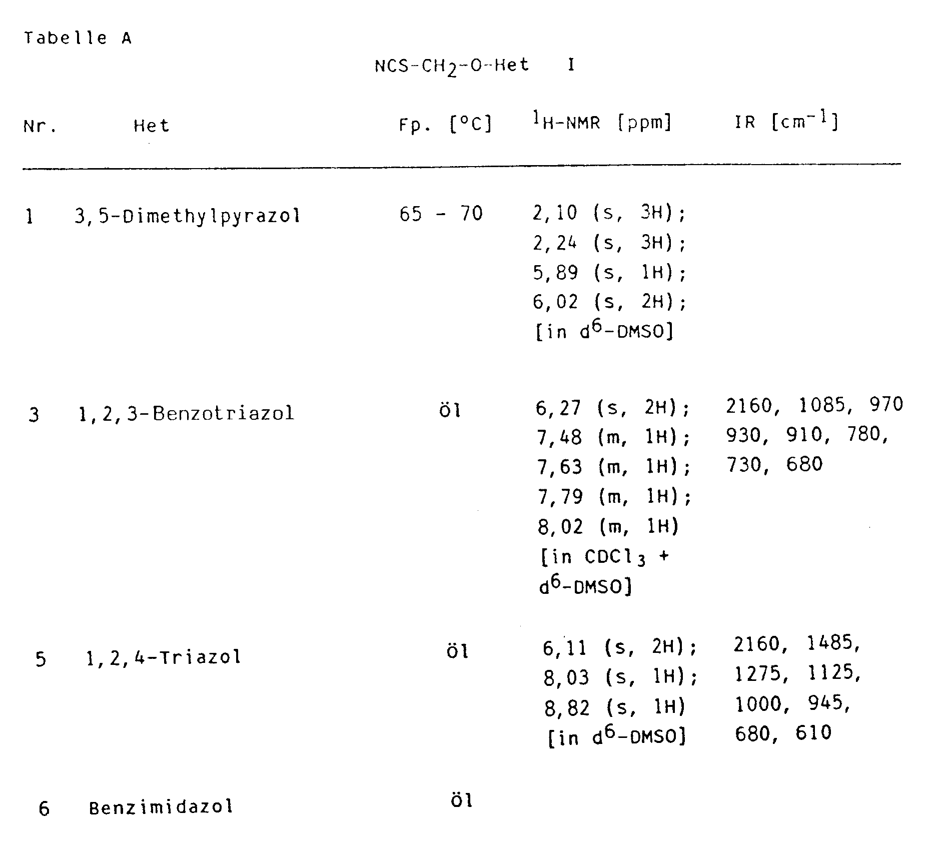

Die neuen Wirkstoffe eignen sich besonders zum Schutz von verschiedenen Materialien gegen den Abbau bzw. die Zerstörung durch Bakterien oder Pilze oder gegen den Befall und Bewuchs durch Mikroorganismen. Materialien, die mit den neuen Wirkstoffen konserviert bzw. mikrozid ausgerüstet werden können, sind beispielsweise Leime und Klebstoffe, Stärkelösungen, Wachsemulsionen, Tonemulsionen, Schlichten, Appreturen, Spinnbäder, Gelatinezubereitungen, Fensterkitt, Fugendichtungsmassen, Kühlschmierstoffe, Bohröle, Treibstoffe, Kunststoffdispersionen, Dispersionsfarben, Textilien, Leder, Rohhäute und Kosmetika. Weiterhin sind die Verbindungen als Schleimbekämpfungsmittel in der Papierindustrie, in Rückkühlwerken und in Luftbefeuchtungsanlagen geeignet.

Des weiteren eignen sich die Verbindungen I zum Schutz folgender Pflanzenarten vor dem Befall durch Mikroorganismen:
Getreide (z.B. Weizen, Gerste, Roggen, Hafer, Reis, Sorghum und Verwandte) Rüben (z.B. Zucker- und Futterrüben); Kern-, Stein- und Beerenobst (z.B. Äpfel, Birnen, Pflaumen, Pfirsiche, Mandeln, Kirschen, Erd-, Him- und Brombeeren); Hülsenfrüchte (z.B. Bohnen, Linsen, Erbsen, Soja); Ölkulturen (z.B. Raps, Senf, Mohn, Oliven, Sonnenblumen, Kokos, Rizinus, Kakao, Erdnüsse); Gurkengewächse (z.B. Kürbis, Gurken, Melonen); Fasergewächse (z.B. Baumwolle, Flachs, Hanf, Jute); Citrusfrüchte (z.B. Orangen, Zitronen, Pampelmusen, Mandarinen); Gemüsesorten (z.B. Spinat, Kopfsalat, Spargel, Kohlarten, Möhren, Zwiebeln, Tomaten, Kartoffeln, Paprika); Lorbeergewächse (z.B. Avocado, Cinnamonum, Kampfer) oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops).

Folgende Mikroorganismen lassen sich beispielsweise mit den erfindungsgemäßen Verbindungen I bekämpfen:
Staphylococcus aureus, Escherichia coli, Klebsielle pneumoniae, Citrobacter freundii, Proteus vulgaris, Pseudomonas aeruginosa, Desulfovibrio desulfuricans, Streptoverticillium rubrireticuli, Aspergillus niger, Aspergillus versicolor, Penicillium funiculosum, Penicillium expansum, Penicillium glaucum, Paecilomyces variotii, Trichoderma viride, Chaetomium globosum, Aspergillus amstelodami, Phoma pigmentovora, Phoma violacea, Aureobasidium pullulans, Saccharomyces cerevisiae, Alternaria tenuis, Stemphylium macrosporoideum, Cladosporium herbarum, Cladosporium resinae, Candida albicans, Trichophyton mentagrophytes, Geotrichum candidans, Monilia sitophila, Scenedesmus quadricauda, Chlorella vulgaris, Nostoc muscorium, Oscillatoria limosa und Anabaena constricta.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser, Trägerstoffe wie natürliche Gesteinsmehle, z.B. Kaoline, Tonerden, Talkum, Kreide und synthetische Gesteinsmehle (z.B. hochdisperse Kieselsäure, Silikate), Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die Formulierungen enthalten im allgemeinen zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % eingesetzt.

Als übliche Anwendungskonzentration wählt man - bezogen auf das Gewicht des zu schützenden Materials - 0,001 bis 5 Gew.-%, bevorzugt 0,01 bis 2 Gew.-% an Wirkstoff; beim Einsatz zur Wasserbehandlung, bei der Erdölförderung, in Bohr- und Schneidölen, Treibstoffen, in Schwimmbädern, Rückkühlwerken, Luftbefeuchtungsanlagen oder in der Papierindustrie sind Wirkstoffmengen von 5 bis 500 ppm ausreichend. Gebrauchsfertige Desinfektionsmittellösungen enthalten z.B. 0,5 bis 10 Gew.-% an Wirkstoff.

Beispiele für solche Zubereitungen sind:
III. eine wäßrige Dispersion aus 20 Gew.-Teilen der Verbindung Nr. 3, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
V. eine in einer Hammermühle vermahlene Mischung aus 80 Gew.-Teilen der Verbindung Nr. 5, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
VI. eine innige Mischung aus 3 Gew.-Teilen der Verbindung Nr. 6 und 97 Gew.-Teilen feinteiligem Kaolin. Dieses Stäubemittel enthält 3 Gew.-% Wirkstoff;
X. eine in einer Hammermühle vermahlene Mischung aus 10 Gew.-Teilen der Verbindung Nr. 5, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Wirkstoffe wirken für sich allein als schaumarme Biozide. Eine bedeutende Steigerung der Wirkung dieser Verbindungen enthaltender biozider Zubereitungen wird erzielt, wenn man ihnen noch Tri-C₆- bis C₁₂-alkylmethylammoniumsalze, vorzugsweise in Mengen von 20 bis 40 Gew.-%, bezogen auf das Gewicht der Verbindungen der allgemeinen Formel I, zusetzt.

Die Wirkstoffe können auch mit anderen bekannten Mikrobiziden gemischt werden. In vielen Fällen erhält man dabei einen synergistischen Effekt, d.h. die mikrobizide Wirksamkeit der Mischung ist größer als die der (addierten) Wirksamkeiten der Einzelkomponenten.

Die Zumischung der bekannten Mikrobizide zu den neuen Substanzen kann in einem Gewichtsverhältnis von 1:100 bis 100:1 erfolgen.

## Patentansprüche

1. N-Thiocyanatomethoxy-Azaheterocyclen der allgemeinen Formel I
NCS-CH₂-O-Het I
in der Het die folgende Bedeutung hat:
- die unsubstituierte 1,2,3-Triazol-1-ylgruppe oder die unsubstituierte 1,2,4-Triazol-1-ylgruppe oder
- die Indazol-1-ylgruppe, die Benzimidazol-1-ylgruppe oder die Benzotriazol-1-ylgruppe, wobei die annellierten Benzolringe dieser Substituenten noch jeweils bis zu drei der folgenden Reste tragen können: Halogen, C₁-C₄-Alkyl, C₁-C₄-Alkoxy oder partiell oder vollständig halogeniertes C₁-C₄-Alkyl,
sowie deren pflanzenverträgliche Salze und Metallkomplexe.

2. Verfahren zur Herstellung der N-Thiocyanatomethoxy-azaheterocyclen I gemäß Anspruch 1, dadurch gekennzeichnet, daß man einen 1-Hydroxy-azaheteroaromaten der Formel IIa
HO-Het IIa
entweder
a) in sein Alkoholat IIb
Het-O^{⊖} IIb
umwandelt und dieses mit Methylenbisthiocyanat umsetzt oder
b) bei Anwesenheit einer Base direkt mit Chlormethylthiocyanat umsetzt oder
c) in sein Alkoholat IIb umwandelt und dieses mit einem Methandihalogenid der Formel III
X-CH₂-Hal III
in der X Chlor, Brom oder Jod bedeutet, zu einem Halogenmethylether der Formel IV
X-CH₂-O-Het IV
umsetzt und diesen mit einem Alkalimetallthiocyanat reagieren läßt.

3. Verwendung der N-Thiocyanatomethoxy-azaheterocyclen I, deren pflanzenverträgliche Salzen oder Metallkomplexe, gemäß Anspruch 1 als Biocide.

4. Biocides Mittel, enthaltend eine biocid wirksame Menge eines N-Thiocyanatomethoxy-azaheterocyclus der Formel I, dessen pflanzenverträglichen Salzes oder Metallkomplexes gemäß Anspruch 1 und einen flüssigen oder festen Trägerstoff.

5. Verfahren zur Bekämpfung von Mikroorganismen, dadurch gekennzeichnet, daß man eine biocid wirksame Menge eines N-Thiocyanatomethoxy-azaheterocyclus I, oder dessen pflanzenverträglichen Salzes oder Metallkomplexen gemäß Anspruch 1 auf die Mikroorganismen oder auf die von dem Befall durch Mikroorganismen bedrohten Gegenstände, Flüssigkeiten oder Suspensionen einwirken läßt.

6. 1-Thiocyanatomethoxy-1,2,4-triazol.

## Claims

1. An N-thiocyanatomethoxyazaheterocycle of the formula I
NCS-CH₂-O-Het I
where Het is unsubstituted 1,2,3-triazol-1-yl or unsubstituted 1,2,4-triazol-1-yl or indazol-1-yl, benzimidazol-1-yl or benzotriazol-1-yl, where the fused benzene rings of these substituents may furthermore each carry up to three of the following radicals: halogen, C₁-C₄-alkyl, C₁-C₄-alkoxy or partially or completely halogenated C₁-C₄-alkyl,
or plant-tolerated salts or metal complexes thereof.

2. A process for the preparation of an N-thiocyanatomethoxyazaheterocycle I as claimed in claim 1, wherein a 1-hydroxyazaheteroaromatic of the formula IIa
HO-Het IIa
is either
a) converted into its alcoholate IIb
Het-O^{⊖} IIb
and the latter reacted with methylene bisthiocyanate, or
b) reacted directly with chloromethyl thiocyanate in the presence of a base, or
c) converted into its alcoholate IIb and the latter reacted with a methylene halide of the formula III
X-CH₂-Hal III
where X is chlorine, bromine or iodine, to give a halomethyl ether of the formula IV
X-CH₂-O-Het IV
and the latter reacted with an alkali metal thiocyanate.

3. Use of an N-thiocyanatomethoxyazaheterocycle I, its plant-tolerated salts or metal complexes as claimed in claim 1 as a biocide.

4. A biocide containing a biocidal amount of an N-thiocyanatomethoxyazaheterocycle of the formula I, its plant-tolerated salt or metal complex as claimed in claim 1 and a liquid or solid carrier.

5. A method for controlling microorganisms, wherein a biocidal amount of an N-thiocyanatomethoxyazaheterocycle I or its plant-tolerated salt or metal complex as claimed in claim 1 is allowed to act on the microorganisms or on the articles, liquids or suspensions threatened by attack by microorganisms.

6. 1-Thiocyanatomethoxy-1,2,4-triazole.

## Revendications

1. N-thiocyanatométhoxy-azahétérocycles de formule générale I
NCS-CH₂-O-Het I
dans laquelle Het a les significations suivantes :
- le groupe 1,2,3-triazol-1-yle non substitué ou le groupe 1,2,4-triazol-1-yle non substitué, ou
- le groupe indazol-1-yle, le groupe benzimidazol-1-yle ou le groupe benzotriazol-1-yle, les noyaux benzéniques condensés de ces substituants pouvant porter en outre deux à trois des restes suivants : halogéno, alkyle en C₁-C₄, alcoxy en C₁-C₄ ou alkyle en C₁-C₄ partiellement ou totalement halogéné,
ainsi que leur sels et complexes métalliques phytocompatibles.

2. Procédé de préparation de N-thiocyanatométhoxy-azahétérocycles I selon la revendication 1 caractérisé en ce que l'on transforme un composé 1-hydroxy-azahétéroaromatique de formule IIa
HO-Het IIa
soit
a) en son alcoolate IIb
Het-O^{⊖} IIb
et on fait réagir celui-ci avec du méthylène-bis-thiocyanate, soit
b) en présence d'une base, on le fait réagir directement avec le chlorométhylthiocyanate, soit
c) on transforme ledit composé en son alcoolate IIb et on fait réagir celui-ci avec un dihalogénure de méthane de formule III
X-CH₂-Hal III
où X représente le chlore, le brome ou l'iode, pour obtenir un halogénométhyléther de formule IV
X-CH₂-O-Het IV
et on fait réagir celui-ci avec un thiocyanate de métal alcalin.

3. Utilisation des N-thiocyanatométhoxy-azahétérocycles I, de leurs sels ou complexes métalliques phytocompatibles, selon la revendication 1 en tant qu'agents biocides.

4. Agent biocide, contenant une quantité biocide active d'un N-thiocyanatométhoxy-azahétérocycle de formule I, ses sels ou complexes métalliques phytocompatibles selon la revendication 1, et un véhicule liquide ou solide.

5. Procédé de lutte contre des micro-organismes, caractérisé en ce que l'on fait agir une quantité biocide active d'un N-thiocyanatométhoxy-azahétérocycle I, ou d'un de ses sels ou complexes métalliques phytocompatibles selon la revendication 1, sur des micro-organismes ou sur des objets, liquides ou suspensions menacés d'être infestés par des micro-organismes.

6. 1-Thiocyanatométhoxy-1,2,4-triazole.
